(19) Europäisches Patentamt
European Patent Office
Office européen des brevets

(11) Publication number: 0 287 819
A1

# (12) EUROPEAN PATENT APPLICATION

(21) Application number: 88104468.9

(51) Int. Cl.4: C07D 413/14

(22) Date of filing: 21.03.88

(30) Priority: 10.04.87 US 36839
02.10.87 US 104702

(43) Date of publication of application:
26.10.88 Bulletin 88/43

(84) Designated Contracting States:
AT BE CH DE ES FR GB IT LI LU NL SE

(71) Applicant: PENNWALT CORPORATION
Pennwalt Building Three Parkway
Philadelphia Pennsylvania 19102(US)

(72) Inventor: Georgiev, Vassil Stefanov
P.O.Box 1032
Penfield New York 14526(US)
Inventor: Mullen, George Byron
1385 Jenks Road
Avon New York 14414(US)

(74) Representative: Kraus, Walter, Dr. et al
Patentanwälte Kraus, Weisert & Partner
Thomas-Wimmer-Ring 15
D-8000 München 22(DE)

## (54) 5-(phenyl or phenoxyalkyl)-3-(2-furanyl or 2-thienyl)-3-(1H-imidazol-1-ylmethyl)-2-methylisoxazolidines.

(57) 5-(Phenyl or phenoxymethyl)-3-(2-furanyl or 2-thienyl)-3-(1H-imidazol-1-ylmethyl)-2-methylisoxazolidines and related derivatives in which one or more hydrogens on the 5-phenyl or phenoxy rings are replaced by halogen, lower alkyl, lower alkoxy, nitro and combinations thereof are useful as antifungal agents.

EP 0 287 819 A1

# 5-(Phenyl or phenoxyalkyl)-3-(2-furanyl or 2-thienyl)-3-(1H-imidazol-1-ylmethyl)-2-methylisoxazolidines

## Background of the Invention

This invention pertains generally to substituted 2-methylisoxazolidines and more specifically to 5-(phenyl or phenoxymethyl)-3-(2-furanyl or 2-thienyl)-3-(1H-imidazol-1-ylmethyl)-2-methylisoxazolidines which are useful as antifungal agents.

## Brief Summary of the Invention

In accordance with this invention there are provided compounds of the formula:

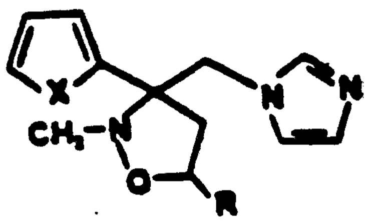

and the pharmaceutically acceptable acid addition salts thereof, in the form of their enantiomers or mixtures of their enantiomers including diastereoisomeric pairs of such enantiomers, wherein;

X is oxygen or sulfur, and

R is selected from phenyl, substituted phenyl, phenoxymethyl, substituted phenoxymethyl, phenylthiomethyl, and (substituted phenyl)thiomethyl groups

wherein the substituents on the substituted phenyl, phenoxymethyl and phenylthiomethyl groups are selected from halogen, nitro, lower alkyl, lower alkoxy and combinations thereof.

## Detailed Description of the Invention

The compounds of this invention are useful as antifungal agents. They have been shown to exert in vitro activity against yeast and systemic mycoses and dermatophytes as determined by broth and agar testing techniques [McGinnis, M.R., Laboratory Handbook of Medical Mycology, Academic Press, N.Y., N.Y. (1980)]. The furanyl compounds prepared in Examples 1 and 3 below were found to have good to moderate inhibitory activity against a broad spectrum of fungi including Trichophyton mentagrophytes, Trichophyton tonsurans, Trichophyton schoenleinii, Epidermophyton floccosum and Candida albicans. The thienyl compounds of Examples 5,6 and 10 were found to have good to moderate activity against Trichophyton rubrum, Aspergilius fumigatus and Candida albicans (minimum inhibitory concentration MIC, of <.2 to 70 μg/ml).

Because of their antifungal activity, the compounds of the invention can be used, for example, in suitable liquid, semisolid or solid carriers in the form of solutions, emulsions, suspensions, dispersions, ointments, aerosols, soaps, detergents, and powders in amounts effective to combat systemic and dermatophylic fungal infections in warm blooded animals (1 to 20 percent active ingredient).

The compounds of this invention are those of the formula:

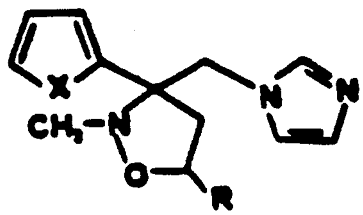

and the pharmaceutically acceptable acid addition salts thereof, in the form of their enantiomers or mixtures of their enantiomers including diastereoisomeric pairs of such enantiomers,
wherein;
x is oxygen or sulfur, and
R represents phenyl, substituted phenyl, phenoxymethyl, substituted phenoxymethyl, phenylthiomethyl and (substituted phenyl)thiomethyl groups. The substituents on the substituted phenyl rings are selected from halogen, nitro, lower alkyl, lower alkoxy and combinations thereof.
By halogen is meant chlorine, bromine, fluorine and iodine, with chlorine and fluorine being preferred. By lower alkyl is meant groups having one to four (1-4) carbons and by lower alkoxy is meant groups having one to six (1-6) carbons which in either case can be a branched or unbranched chain.

The 5-(phenyl, phenoxymethyl or phenylthiomethyl)-3-(2-furanyl or 2-thienyl)-3-(1H-imidazol-1-ylmethyl)-2-methyl-isoxazolidine derivatives are obtained as mixtures of cis-and trans-diastereomers due to the presence of two asymmetric carbon atoms in the isoxazolidine ring. The diastereomeric mixture is conveniently separated by flash chromatography on silica gel using halogenated hydrocarbons (preferably dichloromethane and chloroform), alkanols (preferably methanol and ethanol), ethyl acetate and such, as eluents. The said eluents may be used alone or in combinations, such as the ones comprised of 95-99% halogenated hydrocarbon and 1-5% alkanol by volume. The stereochemistry of the two asymmetric carbon atoms in the isoxazolidine ring may be determined by conventional methods that include x-ray crystallography, nuclear magnetic resonance spectroscopy, circular dichroism and optical rotatory dispersion. Both the cis-and trans-diastereomers are resolvable into their optical enantiomers with (+)-and (-)-optical rotations by standard techniques such as fractional recrystallization of the diastereomeric salts with optically active organic acids such as (+)-and (-)-tartaric acid, (+)-and (-)-dibenzoyltartaric acid and the like.

As illustrated in the following diagram, the furanyl compounds of this invention can be prepared by an initial bromination of 2-acetylfuran 1 (X = O), and reacting the resulting bromo derivatives with imidazole to produce the 1-(2-furanyl)-2-(1H-imidazol-1-yl)ethanone (3). Reaction of compound 3 with N-methylhydroxylamine hydrochloride provides the 1-(2-furanyl)-2-(1H-imidazol-1-yl)-N-methylethanimine N-oxide (4) which is included in the subject matter of our co-pending application Serial Number 900,856 filed August 27, 1986 entitled "α-Substituted Ketonitrone Derivatives" whose disclosure is incorporated herein by reference. The nitrone compound 4 is then treated with styrene or an appropriate styrene (or allyl phenyl ether or allyl phenyl thioether) derivative 5 to give a diastereomeric mixture of the desired cis-and trans-5-(phenyl, phenoxymethyl or phenylthiomethyl)-3-(2-furanyl)-3-(1H-imidazol-1-ylmethyl)-2-methylisoxazolidine 6. The 2-thienyl compounds are prepared similarly by starting with 2-acetylthiophene 1 (X = S).

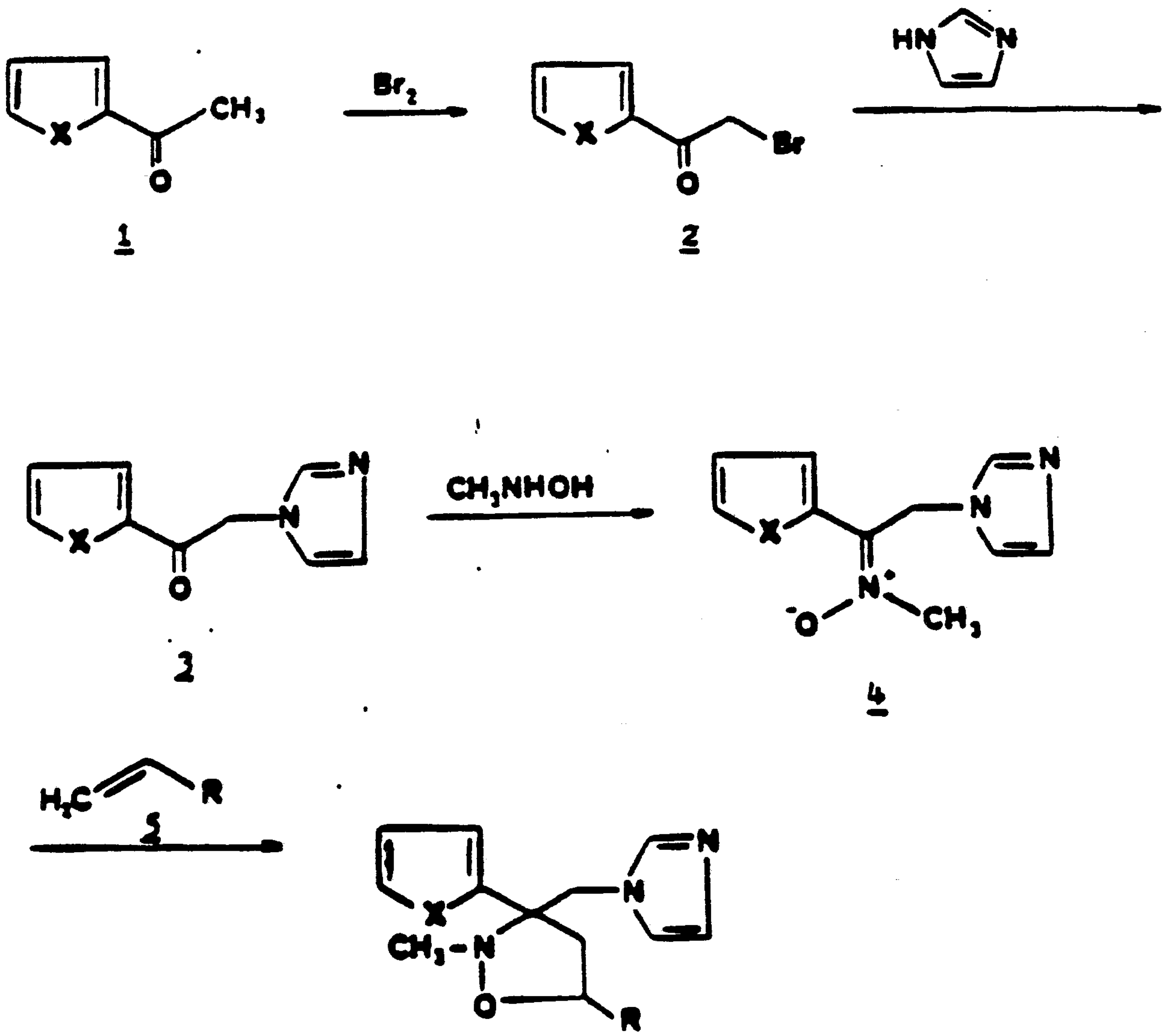


1 2 3 4 5

6

The compounds of this invention are basic and thus can form salts with pharmaceutically acceptable inorganic and organic acids such as, for example, acetic acid, maleic acid, malic acid, fumaric acid, succinic acid, succinamic acid, tartaric acid, citric acid, lactic acid, hydrochloric acid, hydrobromic acid, hydroiodic acid, nitric acid, sulfuric acid and phosphoric acid.

The preparation of the compounds of this invention is further illustrated by the following syntheses of intermediates and in the Examples.

Preparation of 2-Bromo-1-(2-furanyl)ethanone (2)

Bromine (71.14 ml, 1.39 mol) is added dropwise over 1 hour to a solution of 117.59 g (1.07 mol) of 2-acetylfuran (1) in a mixture of 900 ml of ether and 400 ml of 1,4-dioxane, at 0°C under a nitrogen atmosphere. The reaction is warmed to ambient temperature and stirred for 18 hours, then quenched with 1.1 liter of saturated aqueous ammonium chloride and extracted with chloroform. The organic layer is dried

over anhydrous magnesium sulfate and concentrated in vacuo to give a dark oil. Flash-chromatography on neutral silica gel using chloroform as eluent followed by distillation using a Kugelruhr apparatus gives 190.81 g (94%) of compound 2 as a light yellow oil, boiling point 85°C (0.30 mmHg).

Preparation of 1-(2-Furanyl)-2-(1H-imidazol-1-yl)ethanone (3)

To a solution of 8.88 g (0.13 mol) of imidazole and 18.2 ml (0.13 mol) of triethylamine in 180 ml of methanol at 0°C under a nitrogen atmosphere, a solution of 24.66 g (0.13 mol) of 2-bromo-1-(2-furanyl)-ethanone (2) in 50 ml of 1,4-dioxane is added dropwise over 40 min. The reaction mixture is warmed gradually to ambient temperature and stirred for 16 hours, diluted with water and extracted with chloroform. The organic layer is dried over anhydrous magnesium sulfate, concentrated in vacuo, and flash-chromatographed on neutral silica gel using ethyl acetate as eluent to give 20.0 g (87%) of compound 3, melting point 91-94°C (ethyl acetate). Anal. Calcd. for $C_9H_8N_2O_2$: C, 61.36; H, 4.58; N, 15.90. Found: C, 61.40; H, 4.72; N, 15.83.

Preparation of 1-(2-Furanyl)-2-(1H-imidazol-1-yl)-N-methylethanimine N-oxide (4)

Under a nitrogen atmosphere, a mixture of 21.32 g (0.121 mol) of 1-(2-furanyl)-2-(1H-imidazol-1-yl)-ethanone (3), 15.16 g (0.182 mol) of N-methylhydroxylamine hydrochloride, and 14.89 g (0.182 mol) of sodium acetate in 400 ml of ethanol is stirred at ambient temperature for 4 days. Sodium bicarbonate (30.58 g, 0.364 mol) is added and the mixture filtered. The filtrate is concentratedin a 98:2 by volume mixture of chloroform and methanol as eluent to give 8.30 g (33%) of compound 4, melting point 130-133°C (ethyl acetate). Anal. Calcd. for $C_{10}H_{11}N_3O_2$: C, 58.53; H, 5.40; N, 20.48. Found: C, 58.60; H, 5.47; N, 20.49.

Example 1

5-(4-Chlorophenyl)-3-(2-furanyl)-3-(1H-imidazol-1-ylmethyl) -2-methylisoxazolidine (6, X = O, R = $C_6H_4Cl$-4)

A solution of 7.60 g (0.037 mol) of 1-(2-furanyl)-2-(1H-imidazol-1-yl)-N-methylethanimine N-oxide (4) and 7.70 g (0.056 mol) of 4-chlorostyrene in 400 ml of toluene is refluxed for 48 hours under a nitrogen atmosphere. Upon cooling to ambient temperature, the crude cis-and trans-diastereomeric mixture of compound 6 (X = O, R = $C_6H_4Cl$-4) is concentrated in vacuo and flash-chromatographed on neutral silica gel using a 98:2 by volume mixture of chloroform and methanol to give 1.30 g (10%) of isomer A, melting point 99-101°C (ethyl acetate-hexane, 1:1 by volume). Anal. Calcd. for $C_{18}H_{18}ClN_3O_2$: C, 62.88; H, 5.28, Cl, 10.31; N, 12.22. Found: C, 62.90; H, 5.39; Cl, 10.44; N, 12.13. Isomer B (1.10 g, 8.5%) has a melting point of 131-133°C (ethyl acetate-hexane, 1:1 by volume). Anal. Calcd. for $C_{18}H_{18}ClN_3O_2$: C, 62.88; H, 5.28; Cl, 10.31; N, 12.22. Found: C, 62.82; H, 5.28; Cl, 10.46; N, 12.12.

Example 2

3-(2-Furanyl)-3-(1H-imidazol-1-ylmethyl)-2-methyl-5-(3-nitrophenyl)isoxazolidine (6, X = O, R = $C_6H_4NO_2$-3)

Compound 4 (R = $C_6H_4NO_2$-3) is prepared by a method similar to that described in Example 1 by reacting 1-(2-furanyl)-2-(1H-imidazol-1-yl)-N-methylethanimine N-oxide (4) with 3-nitrostyrene. The resulting

cis-and trans-diastereomeric mixture of compound 6 (X = O, R = $C_6H_4NO_2$-3) is flash-chromatographed on neutral silica gel using a 98:2 by volume mixture of chloroform and methanol as eluent. Isomer A has melting point of 120-123°C (ethyl acetate-hexane. 1:1 by volume) Anal. Calcd. for $C_{18}H_{18}N_4O_4$: C, 61.01; H, 5.12; N, 15.81. Found: C, 60.96; H, 5.26; N, 15.88.

Example 3

5-(4-Chlorophenoxymethyl)-3-(2-furanyl)-3-(1H-imidazol-1-ylmethyl)-2-methylisoxazolidine (6, X = O, R = $CH_2OC_6H_4Cl$-4)

Compound 6 (X = O, R = $CH_2OC_6H_4Cl$-4) is prepared by a method similar to that described in Example 1 by reacting 1-(2-furanyl)-2-(1H-imidazol-1-yl)-N-methylethanimine N-oxide (4) with allyl 4-chlorophenyl ether. The resulting cis-and trans-diastereomeric mixture of compound 6 (X = O, R = $CH_2OC_6H_4Cl$-4) is flash-chromatographed on neutral silica gel using a 98:2 by volume mixture of chloroform and methanol as eluent.

Isomer A has melting point of 150-152°C (ethyl acetate). Anal. Calcd. for $C_{19}H_{20}ClN_3O_3$: C, 61.04; H, 5.39; Cl, 9.48; N, 11.24. Found: C, 60.99; H, 5.41; Cl, 9.52; N, 11.21.

Example 4

3-(2-Furanyl)-3-(1H-imidazol-1-ylmethyl)-5-(4-methoxyphenoxymethyl)-2-methylisoxazolidine (6, X = O, R = $CH_2OC_6H_4OCH_3$-4)

Compound 6 (X = O, R = $CH_2OC_6H_4OCH_3$-4) is prepared by a method similar to that described in Example 1 by reacting 1-(2-furanyl)-2-(1H-imidazol-1-yl)-N-methylethanimine N-oxide (4) with allyl 4-methoxyphenyl ether. The resulting cis-and trans-diastereomeric mixture of compound 6 (X = O, R = $CH_2OC_6H_4OCH_3$-4) is flash-chromatographed on neutral silica gel using a 98:2 by volume mixture of chloroform and methanol as eluent. Isomer A has a melting point of 113-115°C (ethyl acetate-hexane, 1:1 by volume). Anal. Calcd. for $C_{20}H_{23}N_3O_4$: C, 65.03; H, 6.28; N, 11.37. Found: C, 65.03; H, 6.28; N, 11.35. Isomer B has a melting point of 118-120°C (ethyl acetate-hexane, 1:1 by volume). Anal. Calcd. for $C_{20}H_{23}N_3O_4$: C, 65.03; H, 6.28; N, 11.37. Found: C, 64.73; H, 6.30; N, 11.22.

Preparation of 2-(1H-Imidazol-1-yl)-1-(2-thienyl) ethanone (3)

Bromine (19.0 ml, 0.371 mol) is added dropwise over 40 minutes to an ice-cold solution of 40.75 g (0.323 mol) of 2-acetylthiophene in 200 ml of ether, under a nitrogen atmosphere. After stirring for 90 min, the reaction is quenched with 100 ml of saturated aqueous ammonium chloride. The layers are separated and the organic layer is washed with 100 ml of water, dried over anhydrous magnesium sulfate and concentrated in vacuo to give a yellow oil (Compound 2), which is dissolved in 100 ml of ether and added over 1 hr to an ice-cold solution of 89.75 g (1.32 mol) of imidazole in 150 ml of methanol, under a nitrogen atmosphere. The reaction is stirred for 22 hrs at ambient temperature, diluted with 1 liter of water and extracted with chloroform (4 x 300 ml). The combined organic layer is dried over anhydrous magnesium sulfate, concentrated in vacuo and flash-chromatographed on neutral silica gel, using a 99:1 by volume mixture of chloroform and methanol as eluent, to give 36.10 g (58%) of compound 3, melting point 87-89°C (ethyl acetate). Anal. Calcd. for $C_9H_8N_2OS$: C, 56.23; H, 4.19; N, 14.57; S, 16.68. Found: C, 56.03; H, 4.28; N, 14.48; S, 16.39.

Preparation of 2-(1H-Imidazol-1-yl)-N-methyl-1-(2-thienyl)ethanimine N-oxide (4)

Under a nitrogen, a mixture of 22.00 g (0.114 mol) of 2-(1H-imidazol-1-yl)-1-(2-thienyl)ethanone (3), 11.49 g (0.138 mol) of N-methylhydroxylamine hydrochloride, and 22.55 g (0.275 mol) of sodium acetate in 250 ml of ethanol is heated to 50°C and stirred for 4 days. The reaction mixture is filtered while hot and the filtrate concentrated in vacuo. Crystallization from ethanol gives 22.62 g (89%) of compound 4, melting point 162-164°C. Anal. Calcd. for $C_{10}H_{11}N_3OS$: C, 54.28; H, 5.01; N, 18.99. Found: C, 54.16; H, 5.06; N, 18.86.

Example 5

3-(1H-Imidazol-1-ylmethyl)-2-methyl-5-phenyl-3-(2-thienyl)isoxazolidine (6, X = S, R = $C_6H_5$)

A suspension of 4.04 g (0.018 mol) of 2-(1H-imidazol-1-yl)-N-methyl-1-(2-thienyl)ethanimine N-oxide (4) and 3.10 ml (1.50 equiv) of styrene in 100 ml of toluene is refluxed for 72 hrs under a nitrogen atmosphere. The reaction mixture is cooled to ambient temperature and filtered. The filtrate, containing the cis-and trans-diastereomeric mixture of compound 6 (R = $C_6H_5$), is concentrated in vacuo and flash-chromatographed on neutral silica gel using a 98:2 mixture of chloroform and methanol as eluent.

Isomer A (2.68 g, 45%) has a melting point of 89-92°C (ethyl acetate). Anal. Calcd. for $C_{18}H_{19}N_3OS$: C, 66.44; H, 5.88; N, 12.91. Found: C, 66.34; H, 5.91; N, 12.92.

Example 6

5-(4-Chlorophenyl)-3-(1H-imidazol-1-ylmethyl)-2-methyl-3-(2-thienyl)isoxazolidine (6, X = S, R = $C_6H_4Cl$-4)

Compound 6 (X = S, R = $C_6H_4Cl$-4) is prepared by a method similar to that described in Example 5 by reacting 2-(1H-imidazol-1-yl)-N-methyl-1-(2-thienyl)ethanimine N-oxide 4 with 4-chlorostyrene. The resulting cis-and trans-diastereomeric mixture of compound 6 (X = S, R = $C_6H_4Cl$-4) is flash-chromatographed on neutral silica gel using ethyl acetate as eluent.

Isomer A has a melting point of 125-127°C (ethyl acetate). Anal. Calcd. for $C_{18}H_{18}ClN_3OS$: C, 60.08; H, 5.04; Cl, 9.85; N, 11.68. Found: C, 60.12; H, 5.05; Cl, 10.10; N, 11.70.

Isomer B has a melting point of 149-152°C (ethyl acetate). Anal Calcd. for $C_{18}H_{18}ClN_3OS$: C, 60.08; H, 5.04; Cl, 9.85; N, 11.68. Found: C, 60.17; H, 5.16; Cl, 10.13; N, 11.69.

Example 7

3-(1H-Imidazol-1-ylmethyl)-2-methyl-5-(3-nitrophenyl)-3-2-thienyl)isoxazolidine 6, X = S, R = $C_6H_4NO_2$-3)

Compound 6 (X = S, R = $C_6H_4NO_2$-3) was prepared by a method similar to that described in Example 5 by reacting 2-(1H-imidazol-1-yl)-N-methyl-1-(2-thienyl)ethanimine N-oxide (4) with 3-nitrostyrene. The resulting cis-and trans-diastereomeric mixture of compound 6 (X = S, R = $C_6H_4NO_2$-3) is flash-chromatographed on neutral silica gel using a 98:2 mixture by volume of chloroform and methanol as eluent. Isomer A has a melting point of 154.5-156°C (ethanol). Anal. Calcd. for $C_{18}H_{18}N_4O_3S$: C, 58.36; H, 4.90; N, 15.12. Found: C, 57.50; H, 4.87; N, 14.92.

Example 8

3-(1H-Imidazol-1-ylmethyl)-2-methyl-5-(phenoxymethyl)-3-(2-thienyl)isoxazolidine 6, X = S, R = $CH_2OC_6H_5$)

Compound 6 (X = S, R = $CH_2OC_6H_5$) was prepared by a method similar to that described in Example 5 by reacting 2-(1H-imidazol-1-yl)-N-methyl-1-(2-thienyl)ethanimine N-oxide (4) with allyl phenyl ether. The resulting cis-and trans-diastereomeric mixture of compound 6 (X = S, R = $CH_2OC_6H_3$) is flash-chromatographed on neutral silica gel using a 98:2 mixture by volume of chloroform and methanol as eluent.

Isomer A has a melting point of 107-109°C (ethyl acetate). Anal. Calcd. for $C_{19}H_{21}N_3O_2S$: C, 64.20; H, 5.95; N, 11.82. Found: C, 64.30; H, 6.09; N, 11.84.

Isomer B has a melting point of 144-146°C (ethyl acetate). Anal. Calcd. for $C_{19}H_{21}N_3O_2S$: C, 64.20; H, 5.95; N, 11.82. Found: C, 64.31; H, 6.18; N, 11.79.

## Example 9

5-[(4-Chlorophenoxy)methyl]-3-(1H-imidazol-1-ylmethyl)-2-methyl-3-(2-thienyl)isoxazolidine (6, X = S, R = $CH_2OC_6H_4Cl$-4)

Compound 6 (X = S, R = $CH_2OC_6H_4Cl$-4) is prepared by a method similar to that described in Example 5 by reacting 2-(1H-imidazol-1-yl)-N-methyl-1-(2-thienyl)ethanimine N-oxide (2) with allyl 4-chlorophenyl ether. The resulting cis-and trans-diastereomeric mixture of compound 6 (X = S, R = $CH_2OC_6H_4Cl$-4) is flash-chromatographed on neutral silica gel using ethyl acetate as eluent.

Isomer A has a melting point of 118-120°C (ethyl acetate). Anal. Calcd. for $C_{19}H_{20}ClN_3O_2S$: C, 58.53; H, 5.17; N, 10.78; S, 8.22. Found: C, 58.69; H, 5.23; N, 10.76; S, 8.50.

Isomer B has a melting point of 95-99°C (ethyl acetate). Anal. Calcd. for $C_{19}H_{20}ClN_3O_2S$: C, 58.53; H, 5.17; Cl, 9.09; N, 10.78; S, 8.22. Found: C, 58.50; H, 5.23; Cl, 9.29; N, 10.68; S, 8.18.

## Example 10

3-(1H-Imidazol-1-ylmethyl)-2-methyl-5-[[(4-methylphenyl)thio]methyl]-3-(2-thienyl)isoxazolidine (6, X = S, R = $CH_2SC_6H_4CH_3$-4)

Compound 6 (X = S, R = $CH_2SC_6H_4CH_3$-4) is prepared by a method similar to that described in Example 5 by reacting 2-(1H-imidazol-1-yl)-N-methyl-1-(2-thienyl)ethanimine N-oxide (4) with allyl 4-methylphenyl sulfide. The resulting cis-and trans-diastereomeric mixture of compound 6 (X = S, R = $CH_2SC_6H_4CH_3$-4) is flash-chromatographed on neutral silica gel using ethyl acetate as eluent.

Isomer A has a melting point of 205-210°C (decomp.) (ethanol) as its monohydrochloride salt. Anal. Calcd. for $C_{20}H_{24}ClN_3OS_2$: C, 56.92; H, 5.73; Cl, 8.40; N, 9.96; S, 15.20. Found: C, 56.88; H, 5.92; Cl, 8.66; N, 9.97; S, 15.30.

Isomer B has a melting point of 159-161°C (ethanol-ether, 1:1 by volume) as its monohydrochloride salt. Anal. Calcd. for $C_{20}H_{24}ClN_3OS_2$: C, 56.92; H, 5.73; Cl, 8.40; N, 9.96; S, 15.20. Found: C, 56.78; H, 5.82; N, 9.92.

## Example 11

5-[[4-Chlorophenyl)thio]methyl]-3-(1H-imidazol-1-ylmethyl-2-methyl-3-(2-thienyl)isoxazolidine (6, X = S, R = $CH_2SC_6H_4Cl$-4)

Compound 6 (X = S, R = $CH_2SC_6H_4Cl$-4) is prepared by a method similar to that described in Example 5 by reacting 2-(1H-imidazol-1-yl)-N-methyl-1-(2-thienyl)ethanimine N-oxide (4) with allyl 4-chlorophenyl sulfide. The resulting cis-and trans-diastereomeric mixture of compound 6 (X = S, R = $CH_2SC_6H_4Cl$-4) is flash-chromatographed on neutral silica gel using a 99:1 mixture by volume of ethyl acetate and methanol as eluent.

Isomer A has a melting point of 101-104°C (ethyl acetate). Ana. Calcd. for $C_{19}H_{20}ClN_3OS_2$: C, 56.21; H, 4.97; Cl, 8.73; N, 10.35; S, 15.79. Found: C, 56.30; H, 5.02; Cl, 8.98; N, 10.37; S, 15.47.

Isomer B has a melting point of 163-173°C (isopropanol) as its monohydrochloride salt.

Example 12

3-(1H-Imidazol-1-ylmethyl)-5-[(4-methoxyphenoxy)methyl]-2-methyl-3-(2-thienyl)isoxazolidine (6, X = S, R = $CH_2OC_6H_4$ $OCH_3$-4)

Compound 6 (X = S, R = $CH_2OC_6H_4OCH_3$-4) can be prepared by a method similar to that described in Example 5 by reacting 2-(1H-imidazol-1-yl)-N-methyl-1-(2-thienyl) ethanimine N-oxide (4) with allyl 4-methoxyphenyl ether.

The compounds of the invention where R is phenyl or lower alkyl phenyl can be prepared according to the method of Example 1 by substituting for styrene,

4-methylstyrene, bp 170-175°C,

and 3-methylstyrene, bp 170-171°C.

The compound of the invention where R is lower alkylphenoxymethyl can be prepared according to the method of Example 3 by substituting for allyl 4-chlorophenyl ether, allyl 4-methylphenyl ether, bp 97-98°C.

Salts of the compounds of the invention can be prepared as known in the art, for example, by dissolving the compound in a 10:1 by volume mixture of ethanol and aqueous acid such as HCl or $HNO_3$, evaporating the solvent, and then recrystallizing the crude salt, for example, from ethanol-ether, methanol-ether, 1:3 by volume or ethanol.

**Claims**

1. A compound of the formula:

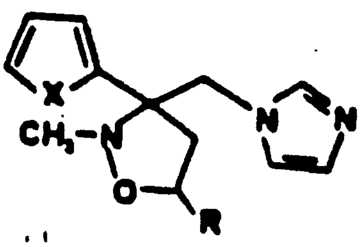

and the pharmaceutically acceptable acid addition salts thereof, in the form of their enantiomers or mixtures of their enantiomers including diastereoisomeric pairs of such enantiomers,

wherein;

X is oxygen or sulfur, and

R is selected from phenyl, substituted phenyl and phenoxymethyl, substituted phenoxymethyl, phenylthiomethyl, and (substituted phenyl)thiomethyl groups wherein the substituents on the substituted phenyl, phenylmethoxy and phenylthiomethyl groups are selected from halogen, nitro, lower alkyl, lower alkoxy and combinations thereof.

2. The compound of claim 1 wherein the compound is 5-(4-chlorophenyl)-3-(2-furanyl)-3-(1H-imidazol-1-ylmethyl)-2-methylisoxazolidine.

3. The compound of claim 1 wherein the compound is 3-(2-furanyl)-3-(1H-imidazol-1-ylmethyl)-2-methyl-5-(3-nitrophenyl)isoxazolidine.

4. The compound of claim 1 wherein the compound is 5-(4-chlorophenoxymethyl)-3-(2-furanyl)-2-(1H-imidazol-1-ylmethyl)-2-methylisoxazolidine.

5. The compound of claim 1 wherein the compound is 3-(2-furanyl)-3-(1H-imidazol-1-ylmethyl)-5-(4-methoxyphenoxymethyl)-2-methylisoxazolidine.

6. The compound of claim 1 wherein the compound is 3-(1H-imidazol-1-ylmethyl)-2-methyl-5-phenyl-3-(2-thienyl)isoxazolidine.

7. The compound of claim 1 wherein the compound is 5-(4-chlorophenyl)-3-(1H-imidazol-1-ylmethyl)-2-methyl-3-(2-thienyl)isoxazolidine.

8. The compound of claim 1 wherein the compound is 3-(1H-imidazol-1-ylmethyl)-2-methyl-5-(3-nitrophenyl)-3-(2-thienyl)isoxazolidine.

9. The compound of claim 1 wherein the compound is 3-(1H-imidazol-1-ylmethyl)-2-methyl-5-(phenoxymethyl)-3-(2-thienyl)isoxazolidine.

10. The compound of claim 1 wherein the compound is 5-[(4-chlorophenoxy)methyl]-3-(1H-imidazol-1-ylmethyl)-2-methyl-3-(2-thienyl)isoxazolidine.

11. The compound of claim 1 wherein the compound is 3-(1H-imidazol-1-ylmethyl)-2-methyl-5-[[(4-methylphenyl)thio]methyl]-3-(2-thienyl)isoxazolidine.

12. The compound of claim 1 wherein the compound is 5-[[(4-chlorophenyl)thio]methyl]-3-(1H-imidazol-1-ylmethyl)-2-methyl-3-(2-thienyl)isoxazolidine.

13. The compound of claim 1 wherein the compound is 3-(1H-imidazol-1-ylmethyl)-5-[(4-methoxyphenoxy)methyl]-2-methyl-3-(2-thienyl)isoxazolidine.

Claims for the following contracting State: ES

1. A process for preparing compounds having the formula:

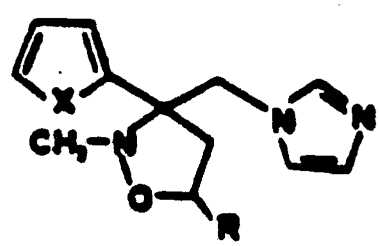

in the form of their enantiomers or mixtures of their enantiomers including diastereoisomeric pairs of such enantiomers,
wherein;
X is oxygen or sulfur, and
R is selected from phenyl, substituted phenyl and phenoxymethyl, substituted phenoxymethyl, phenylthiomethyl, and (substituted phenyl)thiomethyl groups wherein the substituents on the substituted phenyl, phenylmethoxy and phenylthiomethyl groups are selected from halogen, nitro, lower alkyl, lower alkoxy and combinations thereof, comprising, when X = O, reacting 1-(2-furanyl)-2-(1H-imidazol-1-yl)ethanone with N-methylhydroxylamine to form 1-(2-furanyl)-2-(1H-imidazol-1-yl)-N-methylethanimine N-oxide and when X = S, reacting 1-(2-thienyl)-2-(1H-imidazol-1-yl)ethamine with N-methylhydroxylamine to form 1-(2-thienyl)-2-(1H-imidazol-1-yl)-N-methylethanimine N-oxide and then reacting such N-oxides with either styrene, a substituted styrene, an allyl phenyl ether or an allyl phenyl thioether.

2. The process of claim 1 wherein the compound is reacted with an acid so as to form a pharmaceutically acceptable acid addition salt of the compound.

European Patent Office

# EUROPEAN SEARCH REPORT

Application number

EP 88104468.9

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | US - A - 4 397 853 (KAWAKITA)<br>* Column 1, line 24 - column 2, line 35 *<br>-- | 1 | C 07 D 413/14 |
| A | US - A - 3 769 295 (HOYLE)<br>* Formula I *<br>-- | 1 | |
| A | US - A - 3 465 000 (MICETICH)<br>* Claims 1,14 *<br>---- | 1 | |

| TECHNICAL FIELDS SEARCHED (Int Cl 4) |
|---|
| C 07 D 413/00 |

The present search report has been drawn up for all claims

| Place of search | Date of completion of the search | Examiner |
|---|---|---|
| VIENNA | 06-06-1988 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document